(19) Europäisches Patentamt
European Patent Office
Office européen des brevets

(11) Veröffentlichungsnummer: 0 091 041
B1

(12) # EUROPÄISCHE PATENTSCHRIFT

(45) Veröffentlichungstag der Patentschrift: **11.06.86**

(51) Int. Cl.⁴: **C 07 D 333/64**, C 07 D 409/06, A 61 K 31/38, A 61 K 31/44

(21) Anmeldenummer: **83103013.5**

(22) Anmeldetag: **26.03.83**

(54) **Neue vinyloge Carboxamide, Verfahren zu ihrer Herstellung und diese Verbindungen enthaltende Arzneimittel.**

(30) Priorität: **06.04.82 DE 3212752**

(43) Veröffentlichungstag der Anmeldung: **12.10.83 Patentblatt 83/41**

(45) Bekanntmachung des Hinweises auf die Patenterteilung: **11.06.86 Patentblatt 86/24**

(84) Benannte Vertragsstaaten: **AT BE CH DE FR GB IT LI LU NL SE**

(56) Entgegenhaltungen:
**EP - A - 0 025 489**

**JOURNAL OF THE CHEMICAL SOCIETY, PERKIN TRANSACTIONS I, 1980, London L.S.S. REAMONN et al. "Preparation of 2-arylmethylene-5-methylbenzo[b]thiophen-3(2H)-one 1-oxides, 1,1-dioxides, spiroepoxides, 1-oxide spiroepoxides and 1,1-dioxide spiroepoxides", Seiten 1194-1198**
**Chemical Abstracts Band 95, Nr. 3, 20. Juli 1981, Columbus, Ohio, USA L.L. POPOVA et al. "Basicity and structure of azomethines and their structural analogs. 19. Acid properties and structure of heterocyclic aminovinyl ketones and aminovinyl thiones", Seite 604, Spalte 2 Abstract Nr. 24051e**

(73) Patentinhaber: **Dr. Karl Thomae GmbH, Postfach 1755, D-7950 Biberach (Riss) (DE)**

(72) Erfinder: **Engel, Wolfhard, Dr. Dipl.-Chem., Mozartstrasse 13, D-7950 Biberach 1 (DE)**
Erfinder: **Bauer, Eckhart, Dr. Dipl.-Biochem., Nickeleshalde 11, D-7950 Biberach 1 (DE)**
Erfinder: **Trummlitz, Günter, Dr. Dipl.-Chem., Buchenweg 27, D-7951 Warthausen (DE)**
Erfinder: **Danneberg, Peter, Dr., Am St. Jakobsberg 14, D-6531 Ockenheim (DE)**
Erfinder: **Kähling, Joachim, Dr., Haydnweg 8, D-7950 Biberach 1 (DE)**

EP 0 091 041 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der vorliegenden Erfindung sind neue vinyloge Carboxamide der allgemeinen Formel

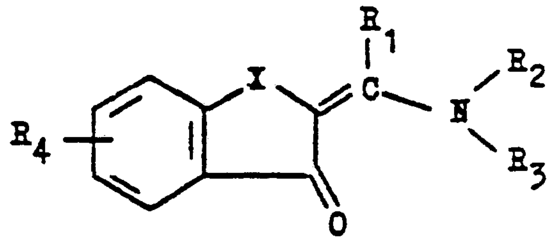

,(I)

Verfahren zu ihrer Herstellung und diese Verbindungen enthaltende Arzneimittel.

Die neuen Verbindungen weisen wertvolle pharmakologische Eigenschaften auf, insbesondere eine Wirkung auf das Zentralnervensystem.

In der obigen allgemeinen Formel I bedeuten:

X die Sulfinyl- oder Sulfonylgruppe,

$R_1$ einen Phenylrest, der durch Halogenatome und/oder Methylqruppen substituiert sein kann, oder einen Pyridinylrest,

$R_2$ ein Wasserstoffatom, eine geradkettige oder verzweigte Alkylgruppe mit 1 bis 4 Kohlenstoffatomen oder eine in 2- oder 3-Stellung durch eine Hydroxy- oder Dimethylaminogruppe substituierte Alkylqruppe mit 2 oder 3 Kohlenstoffatomen,

$R_3$ ein Wasserstoffatom oder eine Alkylqruppe mit 1 bis 3 Kohlenstoffatomen und

$R_4$ die Methylgruppe, ein Wasserstoff- oder Chloratom.

Zur Erläuterung des Erfindungsgegenstandes seien an dieser Stelle folgende Verbindungen als Beispiele genannt:

(E)-2-[(Amino)phenylmethylen]-benzo[b]thiophen-3(2H)-on-1-oxid
(E)-2-[(Methylamino)phenylmethylen-]-benzo[b]thiophen-3(2H)-on-1-oxid
(E)-2-[(Dimethylamino)phenylmethylen]-benzo[b]thiophen-3(2H)-on-1-oxid
(E)-2-[(Amino)-2-fluorphenylmethylen]-benzo[b]thiophen-3(2H)-on-1-oxid
(E)-2-[(Amino)-2-chlorphenylmethylen]-benzo[b]thiophen-3(2H)-on-1-oxid
(E)-2-[[[2-(Dimethylamino)ethyl]amino]phenylmethylen]-benzo[b] thiophen-3(2H)-on-1-oxid
(E)-2-[[2-Hydroxyethyl)amino]phenylmethylen-]-benzo[b] -thiophen-3(2H)-on-1-oxid
(E)-2[[[3-(Dimethylamino)propyl]amino]phenylmethylen]-benzo[b]thiophen-3(2H)-on-1-oxid
(E)-2-[(Amino)-2-methylphenylmethylen]-benzo[b]thiophen-3(2H)-on-1-oxid
(E)-2-[(Ethylamino)phenylmethylen]-benzo[b]thiophen-3(2H)-on-1-oxid
(E)-2[(Amino)-2-bromphenylmethylen]-benzo[b]thiophen-3(2H)-on 1-oxid
(E)-2-[(Amino)-2-iodphenylmethylen]-benzo[b]thiophen-3(2H)-on-1-oxid
(E)-2-[(Amino)-4-chlorphenylmethylen]-benzo[b]thiophen-3(2H)-on-1-oxid
(E)-2-[(Amino)-4-methylphenylmethylen]-benzo[b]thiophen-3(2H)-on-1-oxid
(E)-3-[(Amino)-4-fluorphenylmethylen]-benzo[b]thiophen-3(2H)-on-1-oxid
(E)-2-[(Amino)phenylmethylen]-5-chlorbenzo[b]thiophen-3(2H)-on-1-oxid
(E)-2-[(Amino)phenylmethylen]-6-chlorbenzo[b]thiophen-3(2H)-on-1-oxid
(E)-2-[(Amino)-2-chlorphenylmethylen]-5-chlorbenzo[b]thiophen-3(2H)-on-1-oxid
(E)-2-[(Amino)-2-fluorphenylmethylen]-5-chlorbenzo[b]thiopen-3(2H)-on-1-oxid
(E)-2-[[[2-(Dimethylamino)propyl-]amino] phenylmethylen]-benzo[b]thiophen-3(2H)-on-1-oxid
(E)-2-[[[3-(Dimethylamino)-2-propyl]amino-]phenylmethylen] benzo[b]thiophen-3(2H)-on-1-oxid
(E)-2-[(Propylamino)phenylmethylen]-benzo[b-]thiophen-3(2H)-on-1-oxid
(E)-2-[(Butylamino)phenylmethylen]-benzo[b]thiophen-3(2H)-on-1-oxid
(E)-2-[[(2-Propyl)amino]phenylmethylen]-benzo[b]thiophen-3(2H)-on-1-oxid
(E)-2-[[(2-Methylpropyl)amino]phenylmethylen-]-benzo[b]thiophen-3(2H)-on-1-oxid
(E)-2-[[(1,1-Dimethylethyl)amino] phenylmethylen]-benzo[b]thiophen-3(2H)-on-1-oxid
(E)2-[[(3-Hydroxypropyl)amino]phenylmethylen]-benzo[b]thiophen-3(2H)-on-1-oxid
(E)-2-[(Amino)-3-methylphenylmethyien]-benzo[b]thiophen-3(2H)-on-1-oxid
(E)-2-[(Amino)-4-pyridinylmethylen] -benzo[b]thiophen-3(2H)-on-1-oxid
(E)-2-[(Amino)-3-pyridinylmethylen]-benzo[b]thiophen-3(2H)-on-1-oxid
(E)-2-[(Amino)-2-pyridinylmethylen]-benzo[b]thiophen-3(2H)-on-1-oxid
(E)-2-[(Amino)phenylmethylen-]-benzo[b]thiophen-3(2H)-on-1,1-dioxid

(E)-2-[(Methylamino)phenylmethylen]-benzo[b]thiophen-3(2H)-on-1,1-dioxid
(E)-2-[(Dimethylamino)phenylmethylen]-benzo[b]thiophen-3(2H)-on 1,1-dioxid
(E)-2-[(Amino)-2-fluorphenylmethylen]-benzo[b]thiophen-3(2H)-on-1,1-dioxid
(E)-2-[(Amino)-2-chlorphenylmethylen/-benzo[b]thiophen-3(2H)-on-1,1-dioxid
(E)-2-[[[2-(Dimethylamino)ethyl]amino-]phenylmethylen]-benzo[b] thiophen-3(2H)-on-1,1-dioxid
(E)-2-[[2-Hydroxyethyl)amino]phenylmethylen]-benzo[b]thiophen 3(2H)-on-1,1-dioxid
(E)-2-[[[3-(Dimethylamino)propyl]amino-]phenylmethylen]-benzo[b]thiophen-3(2H)-on-1,1-dioxid
(E)-2-[ Amino)-2-methylphenylmethylei]-benzo[b]thiophen-3(2H)-on-1,1-dioxid
(E)-2-[(Ethylamino)phenylmethylen]-benzo[b]thiophen-3(2H)-on-1,1-dioxid
(E)-2[(Amino)-2-bromphenylmethylen]-benzo[b]thiophen-3(2H)-on-1,1-dioxid
(E)-2-[(Amino)-2-iodphenylmethylen]-benzo[b]thiophen-3(2H)-on-1,1-dioxid
(E)-2-[(Amino)-4-chlorphenylmethyle2]-benzo[b]thiophen-3(2H)-on-1,1-dioxid
(E)-2-[(Amino)-4-methylphenylmethylen]-benzo[b-]thiophen-3(2H)-on-1,1-dioxid
(E)-3-[(Amino)-4-fluorphenylmethylen]-benzo[b]thiophen-3(2H)-on-1,1-dioxid
(E)-2-[(Amino)phenylmethylen]-5-chlorbenzo[b]thiophen-3(2H)-on-1,1-dioxid
(E)-2-[(Amino)phenylmethylen]-6-chlorbenzo[b]thiophen-3(2H)-on-1,1-dioxid
(E)-2-[(Amino)-2-chlorphenylmethylen]-5-chlorbenzo[b]-thiophen-3(2H)-on-1,1-dioxid
(E)-2-[(Amino)-2-fluorphenylmethylen]-5-chlorbenzo[b] thiophen-3(2H)-on-1,1-dioxid
(E)-2-[[[2-(Dimethylamino)propyl]amino]phenylmethylen]-benzo[b]thiophen-3(2H)-on-1,1-dioxid
(E)-2-[[[3-(Dimethylamino)-2-propyl]amino]phenylmethylen]-benzo[b]thiophen-3(2H)-on-1,1-dioxid
(E)-2-[(Propylamino)phenylmethylen]-benzo[b]thiophen-3(2H)-on-1,1-dioxid
(E)-2-[(Butylamino)phenylmethylen-]-benzo[b]thiophen-3(2H)-on-1,1-dioxid
(E)-2-[[(2-Propyl)amino]phenylmethylen]-benzo[b]thiophen-3(2H) -on-1,1-dioxid
(E)-2-[[(2-Methylpropyl)amino]phenylmethylen]-benzo[b] thiophen-3(2H)-on-1,1-dioxid
(E)-2-[[(1,1-Dimethylethyl)amino]phenylmethylen]-benzo[b] thiophen-3(2H)-on-1,1-dioxid
(E)-2-[[(3-Hydroxypropyl)amino]phenylmethylen]-benzo[b]thiophe-n-3(2H)-on-1,1-dioxid
(E)-2-[(Amino)-3-methylphenylmethylen]-benzo[b]thiophen-3(2H)-on-1,1-dioxid
(E)-2-[(Amino)-4-pyridinylmethylen]-benzo[b]thiophen-3(2H)-on-1,1-dioxid
(E)-2-[(Amino)-3-pyridinylmethylen]-benzo[b]thiophen-3(2H)-on-1,1-dioxid
(E)-2-[(Amino)-2-pyridinylmethylen]-benzo[b]thiophen-3(2H)-on-1,1-dioxid

Als bevorzugt gelten die Verbindungen der allgemeinen Formel I, in der
X die Sulfinyl- oder Sulfonylgruppe,
$R_1$ einen gegebenenfalls durch eine Methylgruppe, ein Fluor-, Chlor- oder Bromatom substituierten Phenylrest,
$R_2$ ein Wasserstoffatom, eine Alkylgruppe mit 1 bis 3 Kohlenstoffatomen oder die 2-Hydroxyethylgruppe,
$R_3$ ein Wasserstoffatom oder die Methylgruppe und
$R_4$ die Methylgruppe oder ein Wasserstoff- oder Chloratom bedeuten.

Besonders bevorzugte Verbindungen der allgemeinen Formel 1 sind jedoch diejenigen, in denen
X die Sulfinylgruppe,
$R_1$ einen gegebenenfalls durch eine Methylgruppe, Chloroder Bromatom substituierten Phenylrest,
$R_2$, $R_3$ und $R_4$ jeweils ein Wasserstoffatom bedeuten.

Erfindungsgemäß erhält man die neuen Verbindungen nach folgendem Verfahren:
Oxidation einer Verbindung der allgemeinen Formel

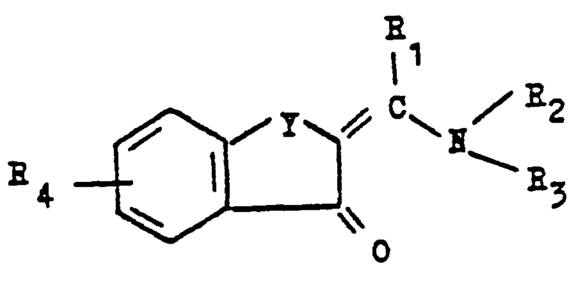
,(II)

in der
$R_1$ bis $R_4$ wie eingangs definiert sind und
Y ein Schwefelatom oder die Sulfinylgruppe bedeutet.

Die Oxidation wird vorzugsweise in einem Lösungsmittel oder Lösungsmittelgemisch, je nach dem verwendeten Oxidationsmittel z.R. in Wasser, Pyridin, Methanol, Ethanol, Aceton, Essigsäure, Ameisensäure, Trifluoressigsäure, Dichlormethan, Trichlormethan oder auch Gemischen davon und bei Temperaturen zwischen -80°C und 120°C, vorzugsweise zwischen -10°C und +80°C, durchgeführt.

Zur Herstellung von Verbindungen der allgemeinen Formel 1, in der X die SO-Gruppe darstellt, wir die Oxidation zweckmäßig mit einem Äquivalent des jeweiligen Oxidationsmittels durchgeführt, z.B. mit Wasserstoffperoxid in Eisessig oder Ameisensäure bei 0 bis 60°C oder in Aceton bei 0 bis 60°C, mit einer Persäure wie Perameisensäure, Peressigsäure oder m-Chlorperbenzoesäure bei 0 bis 60°C, mit Natriummetaperiodat in wäßrigem Methanol oder Ethanol bei 15 bis 30°C, mit Tetrabutylammoniummetaperiodat in Dichlormethan oder 1,2-Di-chlorethan bei 0 bis 60°C, mit N-Bromsuccinimid in Ethanol, mit tert. Rutylhypochlorit in Methanol bei -80 bis -30°C, mit Jodbenzoldichlorid in wäßrigem Pyridin bei 0 bis 50°C, mit Chromsäure in Eisessig oder Aceton bei 0 bis 40°C, mit Wasserstoffperoxid in wäßrigem Methanol oder in Acetonitril und in Gegenwart von Titan(III)chlorid bei 10 bis 60°C, mit Kaliumhydrogenperoxymonosulfat in Methanol oder wäßrigem Methanol bei Temperaturen zwischen 0 und 60°C, mit Natriumbromat in Gegenwart von Cer(IV)-nirat in wäßrigem Acetonitril bei 10 bis 60°C, mit Brom oder Chlor in einem Gemisch von Methylenchlorid und Wasser und in Gegenwart von Natriumoder Kaliumhydrogencarbonat bei 10 bis 60°C, mit Wasserstoffperoxid in Gegenwart von Selendioxid in Methanol und bei Temperaturen zwischen 10 und 60°C, mit Sulfurylchlorid in Methylenchlorid bei -80 bis -40°C; der hierbei primär anfallende Thioether-Chlor-Komplex wird zweckmäßig anschließend mit wäßrigem Ethanol hydrolysiert.

Zur Herstellung von Verbindungen der allgemeinen Formel 1, in der X die $SO_2$-Gruppe darstellt, werden je nach Ausgangsmaterial zweckmäßig ein, zwei oder mehr Oxidationsäquivalente verwendet. Beispielsweise oxidiert man mit Wasserstoffperoxid in Eisessig oder Ameisensdure bei 20 bis 120°C oder in Aceton bei 0 bis 60°C, mit einer Persäure wie Perameisensßure oder m-Chlorperbenzoesdure in Eisessig, Trifluoressigsäure, Trichlormethan oder Dichlormethan bei Temperaturen zwischen 0 und 60°C, mit Salpetersäure in Eisessig bei 0 bis 20°C, mit Chromsäure, Natrium- oder Kaliumpermanganat in Eisessig, Wasser/Schwefelsäure oder in Aceton bei 0 bis 20°C, mit Kaliumhydrogenperoxymonosulfat in Methanol oder wäßrigem Methanol bei 20 bis 60°C.

Bedeutet somit in einer Verbindung der allgemeinen Formel II Y ein Schwefelatom, so wird zur Herstellung einer Sulfinylverbindung der allgemeinen Formel I die Oxidation vorzugsweise mit einem Aquivalent des betreffenden Oxidationsmittels und zur Herstellung einer Sulfonylverbindung der allgemeinen Formel I die Oxidation vorzugsweise mit zwei oder mehr Äquivalenten des betreffenden Oxidationsmittels durchgeführt. Ist hingegen in einer Verbindung der allgemeinen Formel II Y die Sulfinylgruppe, so wird zur Herstellung einer Sulfonylverbindung der allgemeinen Formel I die Oxidation vorzugsweise mit einem oder mehr Äquivalenten des betreffenden Oxidationsmittels durchgeführt.

Die als Ausgangsstoffe verwendeten Thioäther der allgemeinen Formel I sind literaturbekannt (siehe US-PS 4.288.437).

Wie bereits eingangs erwähnt, weisen die neuen Verbindungen der allgemeinen Formel I wertvolle pharmakologische Eigenschaften auf, insbesondere eine Wirkung auf das Zentralnervensystem und antikonvulsive Wirkungen.

Beispielsweise wurden die Verbindungen
A = (E)-2[(Amino)phenylmethylen]-benzo[b]thiophen-3(2H)-on-1-oxid,
B = (E)-2[(Amino)-2-chlorphenylmethylen]-benzo[b]thiophen 3(2H)-on-1-oxid,
C = (E)-2[(Ethylamino)phenylmethylen]-benzo[b]thiophen-3(2H)-on-1-oxid und
D = (E)-2-[(Amino)-2-bromphenylmethylen]-benzo[b]thiophen-3(2H)-on-1,1-dioxid
auf ihre antikonvulsive Wirkung wie folgt untersucht:

**1. Antikonvulsive Wirkung bei Mäusen**

Die antikonvulsive Wirkung wurde als Hemmung des tonischen Extensorkrampfes der Hinterbeine männlicher Mäuse bei maximalem Elektroschock geprüft.

**Methode:**

Versuchstiere waren männliche SPF-Mduse (Chbb: NMRI) im Gewicht von 20-26 g, die bis zu 1 h vor oraler Substanzverabreichung Zugang zu Standardnahrung und Trinkwasser hatten.

Die Versuchsdurchführung erfolgte in Anlehnung an SWINYARD, BROWN und GOODMAN (J. Pharmacol. exp. Ther. 106, 319, [1952]). Die Elektroschock-Apparatur war nach den Angaben von WOODBURY und DAVENPROT (Arch. int. Pharmacodyn. 92, 97, [1952]) hergestellt worden. Die elektrischen Reize wurden über mit Wildleder bezogene und mit 0,9 %iger NaCl-Lösung befeuchtete Stahlkugelelektroden auf die Kopfseiten der Tiere über die Augen gegeben. Es wurde mit Wechselstrom von 50 Hz und 50 mA bei 0,2 sec. Dauer gereizt. Bei allen Kontrolltieren trat dabei ein klonischer und danach ein tonischer Streckkrampf der Extremitäten auf; bei durch Antikonvulsiva geschützten Tieren trat der tonische Streckkrampf nicht auf.

Die zu prüfenden Substanzen wurden in 1 %igem Tyloseschleim suspendiert und in einem Volumen von 0,1 ml/10 g Maus peroral an 10 Mäuse/Dosis verabreicht. Die Tiere wurden 30, 150 und 300 min nach Substanzverabreichung geschockt und die $ED_{50}$-Werte als 50 % der gegen den tonischen Streckkrampf der Hinterextremitdten geschütztehTiere nach dem Verfahren von LITCHFIELD and WILCOXON (J. Pharmacol. exp. Ther. 96, 99, [1949]) oder graphisch ermittelt.

**Ergebnis:**
**Tabelle 1:**

| Substanz | $ED_{50}$ mg/kg p.o. 30 | 150 | 300 Minuten |
|---|---|---|---|
| A | 12,5 | 35 | 88 |
| B | 8,1 | 7,4 | 8,3 |
| C | 17,1 | 61,0 | 93,9 |
| D | 8,6 | 9,3 | 14,6 |

2. **Antikonvulsive Wirkung bei Ratten**
Die antikonvulsive Wirkung wurde als Hemmung des toxischen Extensorkrampfes der Hinterbeine männlicher Ratten bei maximalem Elektroschock geprüft.

**Methode:**
Versuchstiere waren männliche SPF-Ratten (Chbb:THOM) im Gewicht von 100-120 g, die bis zu 1 h vor oraler Substanzverabreichung Zugang zu Standardnahrung und Trinkwasser hatten.

Die Versuchsdurchführung erfolgte in Anlehnung an SWINYARD, BROWN und GOODMAN (J. Pharmacol. exp. Ther. 106, 319, [-1952]). Die Elektroschockapparatur war nach den Angaben von WOODBURY und DAVENPORT (Arch. int. Pharmacodyn. 92, 97 [1952]) hergestellt worden. Die elektrischen Reize wurden über angefeuchtete Stahlkugelelektroden auf die Kopfseiten oberhalb der Augen gegeben. Die Reizparameter waren: Wechselstrom 50 Hz, 50 mA, Reizdauer 0,2 sec.. Bei allen Kontrolltieren trat dabei ein klonischer und danach ein tonischer Streckkrampf der Extremitäten auf; letzterer kann durch wirksame Antikonvulsiva verhindert werden.

Die zu prüfenden Substanzen wurden in 2 %igem Tyloseschleim suspendiert und in einem Volumen von 1 ml/100 g Ratte peroral an 6 Ratten/Dosis mit 1,56, 3,125, 6,25 und 12,5 mg/kg, verabreicht. 1 h danach wurden die Tiere geschockt und die $ED_{50}$ als 50 % der gegen den tonischen Streckkrampf der Hinterextremitäten geschützten Tiere mit den Vertrauensgrenzen für 5 %ige Irrtumswahrscheinlichkeit berechnet.

**Ergebnis:**
**Tabelle 2:** Maximaler Elektrokrampf Ratte, Substanz A.

| Dosis mg/kg p.o | N | geschützte Tiere | % geschützt |
|---|---|---|---|
| 1,56 | 6 | 0 | 0 |
| 3,125 | 6 | 4 | 67 |
| 6,25 | 6 | 5 | 83 |
| 12,5 | 6 | 6 | 100 |

$DE_{50}$ (Vertrauensgrenzen, $p \leq 0,05$) = 3,25 (2,01-5,27) mg/kg p.o.

3. **Verträglichkeit bei Mäusen und Ratten**
Die neue Verbindung A erwies sich als gut verträglich, wie Tabelle 3 in einer orientierenden Toxizitätsprüfung an Mäusen nach peroraler Substanzgabe zeigt.

**Tabelle 3:** Orientierende akute Toxizität bei Mäusen, Substanz A p.o.

| Dosis mg/kg p.o. | N | in 14 Tagen verstorbene Tiere |
|---|---|---|
| 500 | 10 | 0 |
| 1000 | 16 | 1 |
| 1410 | 10 | 0 |
| 2000 | 10 | 2 |
| 4000 | 10 | 1 |

Die $DL_{50}$ für die Maus ist demnach größer als 4000 mg/kg p.o..

Bei Ratten von 200 g Körpergewicht zeigte die Substanz A ebenfalls nur in sehr großen Dosen toxische Wirkungen (Tabelle 4).

**Tabelle 4:** Akute Toxizität Ratte, Substanz A p.o.

| Dosis mg/kg | N | in 14 Tagen verstorbene Tiere | % |
|---|---|---|---|
| 1000 | 10 | 0 | 0 |
| 2000 | 10 | 4 | 40 |
| 4000 | 10 | 7 | 70 |

$DL_{50}$ (Vertrauensgrenzen, $p \leq 0{,}05$) = 2600 (1756-3848) mg/kg p.o.
Die mittlere toxische Dosis beträgt somit bei der Ratte das 800-fache der mittleren therapeutischen Dosis.
Außerdem konnten beim pharmakologischen Screening der untersuchten Substanzen selbst bei den höchsten geprüften Dosen (bis 200 mg/kg p.o.) keine toxischen Nebenwirkungen beobachtet werden.

4. **Untersuchungen zur Gelbfärkung von Fett- und Knorpelgewebe bei der Ratte**

Die bekannte Verbindung (E)-2-[(Amino)phenylmethylen]-benzo-[b]thiophen-3(2H)-on = E (vgl. DE-A-2 931 010.9 oder US-Patent 4 288 437) ist als Arzneimittelwirkstoff durch ihre starke gelbe Eigenfärbung, die an Fett- und Knorpelgewebe abgegeben wird, nur schlecht zu gebrauchen.
Es wurde die Gelbfärbung von Fett- und Knorpelgewebe bei p.o.-Gabe von 50 und 400 mg/kg der Substanz E und der Substanz A 1 x täglich über einen Zeitraum von 5 1/2 Wochen untersucht.

**Methodik:**

Die Substanzen wurden in 1,5 % Tylose unter mehrstündigem Rühren homogen suspendiert.
Die Applikation der Substanzen erfolgte mittels Schlundsonde. Appliziertes Volumen ca. 1,4 ml.
Pro Dosis wurden 9-11 männliche Ratten (Stamm Chbb: THOM (SPF)) mit einem Körpergewicht von 263 ± 10 g eingesetzt.
Zu verschiedenen Zeiten wurden aus den 4 behandelten Gruppen und aus 1 Kontrollgruppe Tiere entnommen und das Nierenfett und Sternum nach Entbluten aus der Bauchaorta freipräpariert.

**Ergebnis:**

Nur bei der 400 mg/kg-Gabe der Substanz E konnte ab dem 17. Tag p.a. zunächst eine schwache, später eine deutliche Gelbfärbung von Nierenfett und Sternum beobachtet werden.

| Behandlungs-dauer Tage | Anzahl der Tiere mit gelb-gefärbtem Nierenfett und Sternum (Anzahl Tiere seziert in Klammer) | | | |
|---|---|---|---|---|
| | Substanz E | | Substanz A | |
| | 50 (mg/kg) | 400 (mg/kg) | 50 (mg/kg) | 400 (mg/kg) |
| 2 | – | 0 (1) | – | 0 (1) |
| 4 | – | 0 (1) | – | 0 (1) |
| 9 | – | 0 (1) | – | 0 (1) |
| 17 | 0 (1) | 1 (1) ganz schwach | 0 (1) | 0 (1) |
| 36 | 0 (1) | 1 (1) deutlich | 0 (1) | 0 (1) |
| 38 | 0 (1) | 1 (1) deutlich | 0 (1) | 0 (1) |
| 39 | 0 (6) | 5 (5) deutlich | 0 (6) | 0 (2) |

Die erfindungsgemäß hergestellten neuen Verbindungen der allgemeinen Formel I eignen sich somit insbesondere zur Behandlung von spastischen Zuständen und der Epilepsie und lassen sich hierzu zur pharmazeutischen Anwendung, gegebenenfalls in Kombination mit anderen Wirksubstanzen, in die üblichen galenischen Zubereitungen wie Tabletten, Dragées, Pulver, Suppositorien oder Suspensionen einarbeiten. Die erforderliche Dosis beträgt hierbei 0,5 bis 5 mg/kg Körpergewicht, vorzugsweise jedoch 1 bis 3 mg/kg Körpergewicht, 2- bis 4-mal täglich.
Die nachfolgenden Beispiele sollen die Erfindung näher erläutern:

**Beispiel 1**

(E)-2-[(Amino)phenylmethylen]-benzo[b]thiophen-3(2H)-on-1-oxid

Die Mischung aus 30,0 g (0,118 Mol) (E)-2-[(Amino)phenyl-methylen] -benzo[b-]thiophen-3(2H)-on, 200 ml Eisessig und 13 5 g (0,119 Mol) 30 proz. wäßriger Wasserstoffperoxid-Lösung wurde 4 Stunden unter Rühren auf 50° C erwärmt und anschließend über Nacht bei Zimmertemperatur stehen gelassen. Nach Zugabe von 100 ml 10 proz. Natriumsulfit-Lösung wurde das Lösungsmittel im Vakuum weitgehend abdestilliert, der verbleibende

Rückstand mit 300 ml Wasser versetzt. Der ausgefallene Feststoff wurde abgenutscht und anschließend aus Ethanol/Essigsäureethylester (1:1 v/v) unter Verwendung von Aktivkohle umkristallisiert. Man erhielt 23,0 g (72 % der Theorie) an hellgelben Kristallen vom F. 246-247°C (Zers.).

**Beispiel 2**

(E)-2-[(Amino)phenylmethylen]-benzo[b]thiophen-3(2H)-on-1,1-dioxid

Die Mischung aus 6,0 g (0,024 Mol) (E)-2-[(Amino)phenyl-methylen]-benzo[b] thiophen-3(2H)-on, 40 ml Eisessig und 5 ml (0,049 Mol) 30 proz. wäßriger Wasserstoffperoxid-Lösung wurde 2 Stunden unter Rühren auf 70°C erwärmt, anschließend nach Zugabe der gleichen Menge Perhydrol-Lösung 6 Stunden unter Rückfluß gekocht. Dann ließ man 14 Stunden bei Zimmertemperatur stehen, versetzte mit 30 ml 10 proz. Natriumsulfit-Lösung und dampfte die Mischung im Wasserstrahlvakuum ein. Der Rückstand wurde in 200 ml Wasser suspendiert und anschließend mit Essigsäureethylester erschöpfend extrahiert.

Die vereinigten Extrakte wurden mit Wasser gewaschen, über Natriumsulfat getrocknet und im Vakuum vom Lösungsmittel befreit. Das so erhaltene Produkt wurde säulenchromatographisch an 300 g Kieselgel unter Verwendung von Dichlormethan/Essigsäureethylester (97:3 v/v) zum Eluieren gereinigt. Durch Eindampfen der geeigneten Fraktionen erhielt man 0,70 g (10 % der Theorie) an hellgelben Kristallen vom F. 198-200°C.

**Beispiel 3**

(E)-2-[(Amino)phenylmethylei]-benzo[b]thiophen-3(2H)-on-1,1-dioxid

3 0 g (0,012 Mol) (E)-2-[(Amino)phenylmethylen]-benzo[b]-thiophen-3(2H)-on wurden in 50 ml Methanol gelöst und mit der Lösung von 7,6 g (0,0124 Mol) "Oxonemonopersulfat" (2 $KHSO_5$ x $KHSO_4$ x $K_2SO_4$; Lieferant: Ventron GmbH, Karlsruhe) in 5 ml Wasser versetzt und anschließend unter Rühren 1 Stunde auf 50°C erwärmt. Die erkaltete Mischung wurde in 300 ml Eiswasser eingerührt und anschließend mit Dichlormethan erschöpfend extrahiert. Man arbeitete wie in Beispiel 2 auf und erhielt nach analoger säulenchromatographischer Reinigung 1,4 g (41 % der Theorie) an hellgelben Kristallen vom F. 199-200°C (Essigsäureethylester), nach Mischschmelzpunkt, Dünnschichtchromatogramm und IR-Spektrum identisch mit einem nach Beispiel 2 erhaltenen Präparat.

**Beispiel 4**

(E)-2-[(Methylamino)phenylmethylen] -benzo[b]thiophen-3(2H)-on-1-oxid
und
(E)-2-[(Methylamino)phenylmethylen] -benzo[b]thiophen-3(2H)-on-1,1-dioxid

Die Mischung aus 6,0 g (0,0224 Mol) (E)-2-[(Methylamino)phenyl-methylen]-benzo[b] thiophen-3(2H)-on, 100 ml Eisessig und 3,5 ml (0,034 Mol) 30 proz. wäßriger Wasserstoffperoxid-Lösung wurde 4 Stunden unter Rühren auf 50°C erwärmt und anschließend über Nacht bei Zimmertemperatur stehen gelassen. Nach Zugabe von 40 ml 10 proz. Natriumsulfit-Lösung wurde im Vakuum eingeengt, der Rückstand in 200 ml Wasser suspendiert. Der ausgefallene Feststoff wurde abgenutscht, anschließend säulenchromatographisch an 300 g Kieselgel unter Verwendung von 1,2-Dichlorethan/Essigsäureethylester (97:3 v/v) in seine Bestandteile zerlegt. Durch Eindampfen der geeigneten Fraktionen erhielt man 2,0 g (33 % der Theorie) an fahlgelbem (E)-2-[(Methylamino)phenylmethylen]-benzo[b]thiophen-3(2H)-on-1-oxid vom F. 205-206°(Zers.) (Ethanol) und 0,10 g (1,5 % der Theorie) an hellgelbem (E)-2-[(Methylamino)phenylmethylen]-benzo[b]thiophen-3(2H)-on-1,1-dioxid vom F. 302-303°C.

**Beispiel 5**

(E)-2-[(Dimethylamino)phenylmethylen] -benzo[b]thiophen-3(2H)-on-1-oxid

Hergestellt analog Beispiel 1 aus (E)-2-[(Dimethylamino)-phenylmethyle2]-benzo[b]thiophen-3(2)-on und Perhydrol in Eisessig.

Ausbeute: 36 % der Theorie,
F: 189-190°C.

**Beispiel 6**

(E)-2-[(Amino)-2-fluorphenylmethylen]-benzo[b]thiophen-3(2H)-on-1-oxid

Hergestellt analog Beispiel 1 aus (E)-2-[(Amino)-2-fluorphenyl-methylen]-benzo[b]thiophen-3(2H)-on und wäßriger Wasserstoffperoxid-Lösung in Eisessig.

Ausbeute: 50 % der Theorie,
F: 220-221°C (Essigsäureethylester).

**Beispiel 7**

(E)-2-[(Amino)-2-chlorphenylmethylen]-benzo[b]thiophen-3(2H)-on-1-oxid

Hergestellt analog Beispiel 1 aus (E)-2-[(Amino)-2-chlorphenyl-methylen]-benzo[b]thiophen-3(2H)-on und wäßriger Wasserstoffperoxid-Lösung in Eisessig.

Ausbeute: 54 % der Theorie,
F: 255-256°C (Zers.). (Methanol:Essigsäureethylester 1:1 v/v).

**Beispiel 8**

(E)-2-[(Amino)-2-bromphenylmethylen]-benzo[b]thiophen-3(2H)-on-1-oxid
und
(E)-2-[(Amino)-2-bromphenylmethylen]-benzo[b]thiophen-3(2H)-on-1,1-dioxid

Hergestellt analog Beispiel 4 aus (E)-2-[(Amino)-2-bromphenyl-methylen]-benzo[b]thiophen-3(2H)-on und wäßriger Wasserstoffperoxid-Lösung in Eisessig.

Ausbeute: 32 % der Theorie an fahlgelbem (E)-2-[(Amino)-2-bromphenylmethylen]-benzo[b]thiophen-3(2H)-on-1-oxid vom F. 243-244°C (Zers.) (Methanol Essigsäureethylester 1:1 v/v) und 18 % der Theorie an hellgelbem (E)-2-[(Amino)-2-brom-phenylmethylen]-benzo[b] thiophen-3(2H)-on-1,1-dioxid vom F. 262-263°C.

**Beispiel 9**

(E)-2-[(Amino)-2-methylphenylmethylen]-benzo[b]thiophen-3(2H)-on-1-oxid

Hergestellt analog Beispiel 1 aus (E)-2-[(Amino)-2-methyl-phenylmethylen]-benzo[b]thiophen-3(2H)-on und wäßriger Wasserstoffperoxid-Lösung in Eisessig.

Ausbeute: 86 % der Theorie,

F: 231-232°C (Zers.) (Ethanol).

**Beispiel 10**

(E)-2-[(Amino)-4-fluorphenylmethylen]-benzo[b]thiophen-3(2H)-on-1-oxid

Hergestellt analog Beispiel 1 aus (E)-2-[(Amino)-4-fluorphenyl-methylen]-benzo[b]thiophen-3(2H)-on und Perhydrol in Eisessig.

Ausbeute: 75 % der Theorie,

F: 235-236°C (Eisessig Ethanol 1:4 v/v).

**Beispiel 11**

(E)-2-[[(2-Hydroxyethyl)amino]phenylmethylen]-benzo[b]thiophen-3(2H)-on-1-oxid

Hergestellt analog Beispiel 1 aus (E)-2-/[](2-Hydroxyethyl)-amino]phenylmethylen]-benzo[b] thiophen-3(2H)-on und Perhydrol in Eisessig.

Ausbeute: 64 % der Theorie,

F: 178-179°C (Ethanol).

**Beispiel 12**

(E)-2-[(Amino)phenylmethylen]-5-chlorbenzo[b]thiophen 3(2H)-on-1-oxid-hemihydrat

und

(E)-2-[(Amino)phenylmethylen]-5-chlorbenzo[b-]thiophen-3(2H)-on-1,1-dioxid

Hergestellt analog Beispiel 4 aus (E)-2-[(Amino)phenylmethylen]-5-chlorbenzo[b]thiophen-3(2H)-on und wäßriger Wasserstoffperoxid-Lösung in Eisessig.

Ausbeute: 53 % der Theorie an (E)-2-[(Amino)phenylmethylen]-5-chlorbenzo[b] thiophen-3(2H)-on-1-oxid-hemihydrat vom F: 251-252°C (Essigsäureethylester) und

18 % der Theorie an (E)-2-[(Amino)phenylmethylen]-5-chlor-benzo[b]thiophen-3(2H)-on-1,1-dioxid vom F. 263-264°C.

**Beispiel 13**

(E)-2-[[[2-(Dimethylamino)ethyl]amino]phenylmethylen]-benzo[b]thiophen-3(2H)-on-1-oxid

Hergestellt analog Beispiel 1 aus (E)-2-[[[2-(Dimethylamino)-ethyl] amino]phenylmethylen-]-benzo[b]thiophen-3-(2H)-on und Perhydrol in Eisessig.

Ausbeute: 29 % der Theorie,

F: 174-176°C (Essigsäureethylester).

**Beispiel 14**

(E)-2-[(Ethylamino)phenylmethylen]-benzo[b]thiophen-3(2H)-on-1-oxid

Hergestellt analog Beispiel 1 aus (E)-2-](Ethylamino)phenyl-methylen]-benzo[b]thiophen-3(2H)-on und wäßriger Wasserstoffperoxid-Lösung in Eisessig

Ausbeute: 11 % der Theorie,

F: 183-185°C.

**Beispiel 15**

(E)-2-[(Amino)-2-chlorphenylmethylen]-5-chlorbenzo[b]thiophen-3(2H)-on-1-oxid

2,5 g (0,0078 Mol) (E)-2-[(Amino)-2-chlorphenylmethylen]-5-chlorbenzo[b]thiophen-3(2H)-on wurden in 150 ml Methanol gelöst, mit der Lösung von 6,15 g (0,01 Mol) "Oxonemonopersulfat" ($KHSO_5$ x $KHSO_4$ x $K_2SO_4$; Lieferant: Ventron GmbH, Karlsruhe) in 50 ml Wasser versetzt und unter Rühren 1 Stunde auf 50°C erwärmt. Die erkaltete Reaktionsmischung wurde in 500 ml Eiswasser eingerührt und anschließend mit Dichlormethan erschöpfend extrahiert. Die vereinigten Extrakte wurden mit Wasser gewaschen, über Natriumsulfat getrocknet und im Vakuum vom Lösungsmittel befreit. Das erhaltene Rohprodukt wurde säulenchromatographisch an 200 g Kieselgel unter Verwendung von Dichlormethan/Essigsäureethylester (97:3 v/v) zum Eluieren gereinigt. Durch Eindampfen der geeigneten Fraktionen erhielt man hellgelbe Kristalle vom F. 248°C (Zers.) (Ethanol),

Ausbeute: 1,5 g (57 % der Theorie).

**Beispiel 16**

(E)-2-[(Amino)phenylmethylen]-5-methylbenzo[b]thiophen-3(2H)-on-1-oxid-monohydrat

Hergestellt analog Beispiel 1 aus (E)-2-[(Amino)phenyl-methylen]-5-methylbenzo[b] thiophen-3(2H)-on und wäßriger

Wasserstoffperoxid-Lösung in Eisessig.

Ausbeute: 45 % der Theorie,

F: 233-235°C (Essigsäureethylester).

**Beispiel 17**

(E)-2-[(Amino)phenylmethylen]-benzo[b]thiophen-3(2H)-on-1-oxid

Hergestellt analog Beispiel 15 aus (E)-2-[(Amino)phenyl-methylen]A-benzo[b]thiophen-3(2H)-on und "Oxonemonopersulfat", jedoch unter Verwendung von Ethanol an Stelle von Methanol. Ausbeute: 66 % der Theorie,

F: 246-247°C (Ethanol).

**Beispiel 18**

(E)-2-[(Amino)phenylmethylenl -benzo[b]thiophen-3(2H)-on-1-oxid

Zu der Lösung von 2,53 g (0,01 Mol) (E)-2-[(Amino)phenyl-methylen]-benzo[b]thiophen-3(2H)-on in 150 ml Methanol gab man die Lösung von 2,246 g (0,0105 Mol) Natriummetaperiodat in 10 ml Wasser und rührte die Mischung 15 Stunden bei Zimmertemperatur. Man entfernte das ausgeschiedene Natriumiodat durch Filtration, verdünnte das Filtrat mit 300 ml Wasser und extrahierte es erschöpfend mit Dichlormethan. Die vereinigten Extrakte wurden einmal mit Wasser gewaschen, über Natriumsulfat getrocknet und im Vakuum eingedampft. Der Rückstand wurde aus Ethanol/Essigsäureethylester unter Verwendung von Aktivkohle umkristallisiert und ergab 2,28 g (85 % der Theorie) an fahlgelben Kristallen vom F. 246-247°C (Zers.).

**Beispiel 19**

(E)-2-[(Amino)phenylmethylen]-benzo[b]thiophen-3(2H)-on-1,1-dioxid

Zu einer Lösung von 3,61 g (13,4 mMol) (E)-2-[(Amino)phenyl-methylen]-benzo[b]thiophen-3(2H)-on-1-oxid in 30 ml Eisessig gab man portionsweise eine Lösung von 2,1 g (13,4 mMol) Kaliumpermanganat in 15 ml Wasser. Die Temperatur hielt man dabei auf 15-20°C. Man rührte noch 1 Stunde bei Zimmertemperatur, verdünnte mit 200 ml Wasser, reduzierte mit überschüssiger Natriumhydrogensulfit-Lösung den ausgefallenen Braunstein und extrahierte die Mischung erschöpfend mit Methylenchlorid. Die vereinigten Extrakte wurden mit Wasser gewaschen, über Natriumsulfat getrocknet und im Vakuum eingeengt. Den Rückstand kristallisierte man aus Ethanol]Essigsäureethylester unter Verwendung von Aktivkohle um.

Ausbeute: 2,5 g (65 % der Theorie),

F: 198-200°C (Zers.)

**Beispiel 20**

(E)-2-[(Amino)phenylmethylen]-benzo[b]thiophen-3(2H)-on-1-oxid

1,18 g (4,66 mMol) (E)-2-[(Amino)phenylmethylen]benzo[b]-thiophen-3(2H)-on wurden in 50 ml Methanol gelöst und mit 0,9 g (4,94 mMol) N-Bromsuccinimid versetzt. Man rührte 15 Stunden bei Zimmertemperatur, verdünnte mit 500 ml 60°C heißem Wasser und dekantierte von dem kristallisierenden Rückstand ab. Nach dem Umkristallisieren aus Ethanol erhielt man 0,83 g (66 % der Theorie) an fahlgelben Kristallen vom F. 245-247°C (Zers.).

**Beispiel 21**

(E)-2-[(Amino)-4-fluorphenylmethylen]-benzo[b]thiophen-3(2H)-on-1-oxid

2,61 g (9,61 mMol) (E)-2-[(Amino)-4-fluorphenylmethylen]-benzo[b-]thiophen-3(2H)-on wurden in 50 ml Dichlormethan gelöst, auf -70°C abgekühlt und dann mit einer Lösung von 1,5 g 11,1 mMol) Sulfurylchlorid in 5 ml Dichlorethan tropfenweise versetzt. Nach 15 Stunden wurde mit 20 ml 95 proz. Ethanol versetzt und durch Entfernen des Kühlbades auf Zimmertemperatur erwärmt. Man neutralisierte mit wäßriger Natriumcarbonat-Lösung, trocknete die Dichlormethan-Schicht mit Natriumsulfat, dampfte das Lösungsmittel im Vakuum ab und kristallisierte den Rückstand aus Eisessig]Ethanol (1:4 v/v) um.

Ausbeute: 2,24 g (81 % der Theorie),

F: 235-236°C.

**Beispiel 22**

(E)-2-[(Amino)phenylmethylen]-benzo[b]thiophen-3(2H)-on-1-oxid

Eine Mischung aus 0,51 g (2 mMol) (E)-2-[(Amino)phenyl-methylen]-benzo[b]thiophen-3(2H)-on, 378 mg (2,5 mMol) Natriumbromat und 28 mg (0,051 mMol) Ammoniumcer(IV)-nitrat in 10 ml wässerigem Acetonitril (7:3 v]v) wurde 5 Stunden bei Zimmertemperatur kräftig gerührt. Die Mischung wurde mit 200 ml Wasser verdünnt und das auskristallisierte Produkt abgenutscht. Nach Umkristallisieren aus Ethanol]Essigsäureethylester (1:1 v/v) unter Verwendung von Aktivkohle erhielt man 0,49 g (91 % der Theorie) an fahlgelben Kristallen vom F. 246-247°C (Zers.).

**Beispiel 23** (E)-2-[(Amino)phenylmethylen]-benzo[b]thiophen-3(2H)-on-1-oxid

Ein 250 ml Dreihalsrundkölbchen wurde mit 30 ml Dichlormethan, 2,53 g (10 mMol) (E)-2-[(Amino)phenylmethylen]-benzo[b]-thiophen-3(2H)-on und 20 ml einer 10 proz. Kaliumhydrogencarbonat-Lösung beschickt. Unter Rühren tropfte man innerhalb von 1 Stunde die Lösung von 1,6 g (10 mMol) Brom in 20 ml Dichlormethan ein und hielt anschließend weitere 4 Stunden bei Zimmertemperatur. Nach dieser Zeit war die rotbraune Bromfarbe verschwunden. Man trennte die untere organische Schicht ab, zog die wässerige Phase noch zweimal mit je 50 ml Methylenchlorid aus, vereinigte die organischen Extrakte und trocknete sie über Natriumsulfat. Der nach dem Abdampfen des Lösungsmittels verbleibende Rückstand wurde aus Ethanol umkristallisiert. Ausbeute: 2,56 g (95 % der Theorie),

F: 246-247°C (Zers.)

Verwendete man unter sonst gleichen Bedingungen an Stelle von Brom die äquivalente Menge Chlor als Oxidationsmittel, so war die Umsetzung nach 20 Minuten beendet.

Ausbeute: 88 % der Theorie,

F: 245-247°C (Zers.) (Ethanol).

**Beispiel 24**

(E)-2-[(Amino)phenylmethylen-]-benzo[b]thiophen-3(2H)-on-1-oxid

Zu der Lösung von 2,53 g (10 mMol) (E)-2-[(Amino)phenyl-methylen]-benzo[b]thiophen-3(2H)-on in 20 ml Methanol tropfte man unter Rühren die Lösung von 1,1 ml (l0,8 mMol) einer 30 proz. Wasserstoffperoxid-Lösung und von 1,1 g (10 mMol) Selendioxid in 5 ml Wasser. Dabei sollte die Reaktionstemperatur +25°C nicht übersteigen. Nach 20 Minuten verdünnte man mit 100 ml Wasser, nutschte das Reaktionsprodukt ab und kristallisierte es aus Ethanol/Essigsäureethylester (1:1 v/v) unter Verwendung von Aktivkohle um.

Ausbeute: 2,32 g (86 % der Theorie),
F: 246-247°C (Zers.)

**Beispiel 25**

(E)-2-[(Amino)phenylmethylen]-benzo[b]thiophen-3(2H)-on-1-oxid

7,6 g (0,03 Mol) (E)-2-[(Amino)phenylmethylen]-benzo[b]thiophen-3(2H)-on und 13,75 g (0,03 Mol) Tetrabutylammoniumperiodat (hergestellt aus Tetrabutylammoniumhydrogensulfat und Natriummetaperiodat in Wasser) in 50 ml Dichlormethan wurden ca. 2 Stunden unter Rückfluß gekocht. Nach Beendigung der Reaktion, die durch dünnschichtchromatographische Untersuchungen verfolgt wurde, ließ man erkalten und filtrierte die Mischung über eine Kieselgelsäule (200 g), zuletzt unter Verwendung von Dichlormethan]Ethylacetat (97:3 v/v) zum Eluieren. Durch Eindampfen der geeigneten Fraktionen und anschließendes Umkristallisieren aus Ethanol erhielt man 5,8 g (72 % der Theorie) an fahlgelben Kristallen vom F. 246-247°C (Zers.).

**Beispiel 26**

(E)-2[(Ethylamino)phenylmethylen-]-benzo[b]thiophen-3(2H)-on-1-oxid

Zu der Lösung von 1,97 g (0,007 Mol) (E)-2-[(Ethylamino)-phenylmethylen]-benzo[b]thiophen-3(2H)-on und 10 ml einer 16 proz. wäßrigen Lösung von Titan(III)-chlorid in 100 ml Methanol und 20 ml Wasser tropfte man unter gutem Rühren 3,2 ml (0,031 Mol) einer 30 proz. wäßrigen Wasserstoffperoxid-Lösung, gelöst in 15 ml Methanol. Der Verlauf der Umsetzung wurde dünnschichtchromatographisch verfolgt. Nach Beendigung der Umsetzung verdünnte man mit 300 ml Wasser, saugte den entstandenen Niederschlag ab und kristallisierte ihn zweimal aus Ethanol um. Man erhielt 1,75 g (84 % der Theorie) an fahlgelben Kristallen vom F. 183-185°C.

**Beispiel 27**

(E)-2-[ Amino)-2-bromphenylmethylen]-benzo[b]thiophen-3(2H)-on-1,1-dioxid

1,74 g (5 mMol) (E)-2-[(Amino)-2-bromphenylmethylen]-benzo[b]thiophen-3(2H)-on wurden in einem Gemisch aus 50 ml Dichlormethan und 5 ml Eisessig gelöst, dann auf -10°C abgekühlt und portionsweise mit 1,71 g (5,5 mMol) Benzyltriethylammoniumpermanganat versetzt. Man rührte eine weitere Stunde bei -10°C, verdünnte mit 300 ml Dichlormethan, ließ absitzen und filtrierte über Aktivkohle. Das Filtrat wurde zweimal mit Wasser, zweimal mit 10 proz. wäßriger Natriumhydrogensulfit-Lösung, abermals mit Wasser und zuletzt mit gesättigter Natriumhydrogensulfit-Lösung gewaschen, über Natriumsulfat getrocknet, schließlich im Vakuum eingedampft. Der hellgelbe, kristalline Rückstand schmolz nach dem Auskochen mit Wasser, Tetrahydrofuran und Ether bei 243-244°C (Zers.). Ausbeute: 0,98 g (54 % der Theorie).

**Beispiel 28**

(E)-2 [(Amino)phenylmethylen]-benzo[b]thiophen-3(2H)-on-1-oxid

Zu der Suspension von 127,0 g (0,501 Mol) (E)-2-[ Amino)phenylmethylen]-benzo[b] thiophen-3(2H)-on in 4 l Ethanol gab man portionsweise die Lösung von 107,0 g (0,500 Mol) Natriummetaperiodat in 800 ml Wasser und rührte die Mischung 12 Stunden bei Zimmertemperatur. Anschließend gab man nochmals 54,0 g (0,252 Mol) Natriummetaperiodat, gelöst in 500 ml Wasser, sowie 2 1 Ethanol zu und rührte weitere 8 Stunden bei Zimmertemperatur.

Dann wurde die Mischung in 6 l Wasser eingerührt und das ausgefallene Produkt abgenutscht. Den Filterrückstand wusch man dreimal mit je 200 ml 40°C warmen Wassers und einmal mit 200 ml kaltem Methanol und kristallisierte ihn dann aus möglichst wenig Dimethylformamid um. Das so erhaltene Produkt wurde in 200 ml eines Gemisches aus Methanol und Chloroform (1:1 v]v) gelöst, mit 3 g Tierkohle behandelt und heiß filtriert. Das Filtrat wurde auf ein Viertel seines ursprünglichen Volumens eingedampft und über Nacht bei Zimmertemperatur aufbewahrt. Der Niederschlag wurde dann abgenutscht und mit Diethylether nachgewaschen. Man trocknete bei 110°C im Vakuum und erhielt 98,0 g (73 % der Theorie) an fahlgelben Kristallen vom F. 246-247°C (Zers.).

**Beispiel 29**

(E)-2-[(Amino)-2-pyridinylmethylen]-benzo[b]thiophen-3(2H)-on-1-oxid

Hergestellt analog Beispiel 28 aus (E)-2-[(Amino)-2-pyridinvl-methylen-]-benzo[b]thiophen-3(2H)-on und Natriummetaperiodat. Ausbeute: 33 % der Theorie.

F. 186-187°C (Essigsäureethylester).

**Beispiel 30**

(E)-2-[(Amino)-4-pyridinylmethylen]-benzo[b]thiophen-3(2H)-on-1-oxid

Hergestellt analog Beispiel 29 aus (E)-2-[(Amino)-4-pyridinyl-methylen]-benzo[b]thiophen-3(2H)-on und Natriummetaperiodat. Ausbeute: 80 % der Theorie.

F. 259-260°C (Essigsäureethylester).

**Beispiel I**

Tabletten enthaltend 50 mg (E)-2[(Amino)phenylmethylen]-benzo[b]thiophen-3(2H)-on-1-oxid

Zusammensetzung:
Wirkstoff 50 mg
Milchzucker 148 mg
Kartoffelstärke 60 mg
Magnesiumstearat 2 mg
260 mg

Man rührt in eine 10%ige Kartoffelstärkelösung die Wirksubstanz und den Milchzucker und granuliert das Gemenge durch ein Sieb der Maschenweite 1,5 mm, trocknet das Granulat und reibt es erneut durch dasselbe Sieb, man fügt das Magnesiumstearat zu und verpresst das Granulat zu Tabletten.

Gewicht der Tablette: 260 mg.

**Beispiel II**

Dragées enthaltend 50 mg (E)-2[(Methylamino)phenylmethylen]-benzo[b]thiophen-3(2H)-on-1-oxid

Man stellt analog zu Beispiel I ein Granulat her, welches zu Dragée-Kernen verpreßt wird. Die Kerne werden mit einer Hülle versehen, die im wesentlichen aus Zucker und Talk besteht.

Die Hülle wird anschließend mit Bienenwachs poliert.

Gewicht des Kerns: 260 mg

Gewicht des Dragées: 200 mg.

## Patentansprüche

1.) Vinyloge Carboxamide der allgemeinen Formel

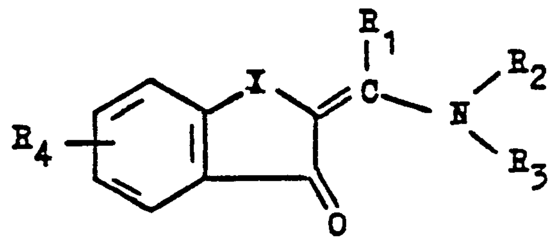

,(I)

in der

X die Sulfinyl- oder Sulfonylgruppe,

$R_1$ einen Phenylrest, der durch Halogenatome und/oder Methylgruppen substituiert sein kann, oder einen Pyridinylrest,

$R_2$ ein Wasserstoffatom, eine geradkettige oder verzweigte Alkylgruppe mit 1 bis 4 Kohlenstoffatomen oder eine in 2- oder 3-Stellung durch eine Hydroxy- oder Dimethylaminogruppe substituierte Alkylgruppe mit 2 oder 3 Kohlenstoffatomen,

$R_3$ ein Wasserstoffatom oder eine Alkylgruppe mit 1 bis 3 Kohlenstoffatomen und

$R_4$ die Methylgruppe, ein Wasserstoff- oder Chloratom bedeuten.

2.) Vinyloge Carboxamide der allgemeinen Formel I gemäß Anspruch 1, in der

X die Sulfinyl- oder Sulfonylgruppe,

$R_1$ einen gegebenenfalls durch eine Methylgruppe, ein Fluor-, Chlor- oder Bromatom substituierten Phenylrest,

$R_2$ ein Wasserstoffatom, eine Alkylgruppe mit 1 bis 3 Kohlenstoffatomen oder die 2-Hydroxyethylgruppe,

$R_3$ ein Wasserstoffatom oder die Methylgruppe und

$R_4$ die Methylgruppe, ein Wasserstoff- oder Chloratom bedeuten.

3.) Vinyloge Carboxamide der allgemeinen Formel I gemäß Anspruch 1, in der

X die Sulfinylgruppe,

$R_1$ die Phenyl-, 2-Chlorphenyl- oder 2-Bromphenylgruppe,

$R_2$, $R_3$ und $R_4$ je ein Wasserstoffatom bedeuten.

4.) (E)-2[(Amino)phenylmethylen]-benzo[b]thiophen-3(2H)-on-1-oxid.

5.) Arzneimittel, enthaltend eine Verbindung gemäß den Ansprüchen 1 bis 4 neben gegebenenfalls einem oder mehreren inerten Trägerstoffen und]oder Verdünnungsmitteln.

6.) Verwendung einer Verbindung gemäß den Anspruch 1 bis 4 zur Herstellung eines antikonvulsiv wirkenden Arzneimittels.

7.) Verfahren zur Herstellung von neuen vinylogen Carboxamiden der allgemeinen Formel

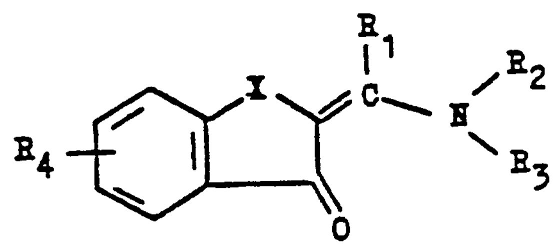

,(I)

in der

X die Sulfinyl- oder Sulfonylgruppe,

$R_1$ einen Phenylrest, der durch Halogenatome und/oder Methylgruppen substituiert sein kann, oder einen Pyridinylrest,

$R_2$ ein Wasserstoffatom, eine geradkettige oder verzweigte Alkylgruppe mit 1 bis 4 Kohlenstoffatomen oder

eine in 2- oder 3-Stellung durch eine Hydroxy- oder Dimethylaminogruppe substituierte Alkylgruppe mit 2 oder 3 Kohlenstoffatomen,

$R_3$ ein Wasserstoffatom oder eine Alkylgruppe mit 1 bis 3 Kohlenstoffatomen und

$R_4$ die Methylgruppe, ein Wasserstoff- oder Chloratom bedeuten, dadurch gekennzeichnet, daß eine Verbindung der allgemeinen Formel

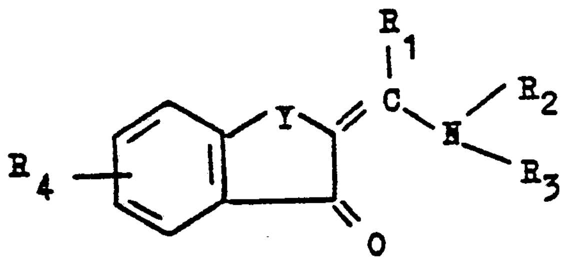

,(II)

in der

$R_1$ bis $R_4$ wie eingangs definiert sind und

Y ein Schwefelatom oder die Sulfinylgruppe darstellt, oxidiert wird.

8.) Verfahren gemdß Anspruch 7, dadurch gekennzeichnet, daß die Umsetzung in einem Lösungsmittel und bei Temperaturen zwischen -80 und 120° C, vorzugsweise jedoch bei Temperaturen zwischen -10 und 80° C, durchgeführt wird.

9.) Verfahren gemäß den Ansprüchen 7 und 8 zur Herstellung von Verbindungen der allgemeinen Formel I, in der X die Sulfinylgruppe bedeutet, dadurch gekennzeichnet, daß eine Verbindung der allgemeinen Formel II, in der Y ein Schwefelatom darstellt, mit einem Äquivalent eines Oxidationsmittels oxidiert wird.

10.) Verfahren gemäß den Ansprüchen 7 und 8 zur Herstellung von Verbindungen der allgemeinen Formel I, in der X die Sulfonylgruppe bedeutet, dadurch gekennzeichnet, daß eine Verbindung der allgemeinen Formel II, in der Y die Sulfinylgruppe darstellt, mit einem oder mehr Äquivalenten eines Oxidationsmittels, eine Verbindung der allgemeinen Formel II, in der Y ein Schwefelatom darstellt, mit zwei oder mehr Äquivalenten eines Oxidationsmittels oxidiert wird.

**Claims**

1. Vinylogous carboxamides of general formula

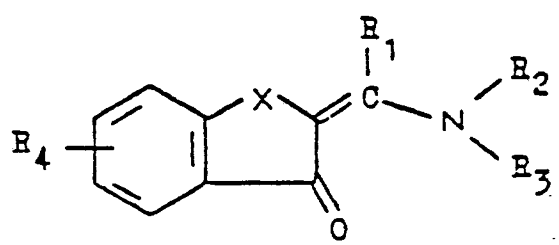

(I)

wherein

X represents the sulphinyl or sulphonyl group, $R_1$ represents a phenyl group which may be substituted by halogen atoms and/or methyl groups, or a pyridinyl group,

$R_2$ represents a hydrogen atom, a straightchained or branched alkyl group with 1 to 4 carbon atoms or an alkyl group with 2 or 3 carbon atoms substituted in the 2- or 3-position by a hydroxy or dimethylamino group,

$R_3$ represents a hydrogen atom or an alkyl group with 1 to 3 carbon atoms, and

$R_4$ represents a methyl group or a hydrogen or chlorine atom.

2. Vinylogous carboxamides of general formula I as claimed in claim 1, wherein

X represents a sulphinyl or sulphonyl group, $R_1$ represents a phenyl group optionally substituted by a methyl group or by a fluorine, chlorine or bromine atom,

$R_2$ represents a hydrogen atom, an alkyl group with 1 to 3 carbon atoms or a 2-hydroxyethyl group,

$R_3$ represents a hydrogen atom or a methyl group and

$R_4$ represents a methyl group or a hydrogen or chlorine atom.

3. Vinylogous carboxamides of general formula I as claimed in claim 1, wherein

X represents a sulphinyl group,

$R_1$ represents a phenyl, 2-chlorophenyl or 2-bromophenyl group,

$R_2$, $R_3$ and $R_4$ each represent a hydrogen atom.

4. (E)-2-[(Amino)phenylmethylene]-benzo[b]thiophen-3(2H)-one-1-oxide.

5. Pharmaceutical compositions containing a compound as claimed in claims 1 to 4, optionally together with one or more inert carriers and/or diluents.

6. Use of a compound as claimed in claims 1 to 4 for the preparation of a pharmaceutical composition having an anticonvulsive effect.

7. Process for the preparation of new vinylogous carboxamides of general formula

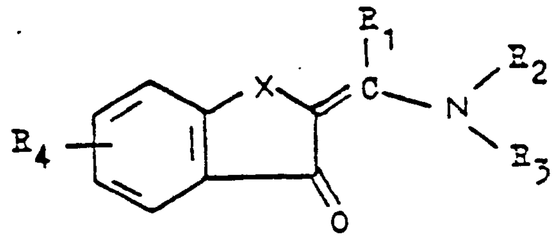

(I)

wherein

X represents the sulphinyl or sulphonyl group, $R_1$ represents a phenyl group which may be substituted by halogen atoms and/or methyl groups, or a pyridinyl group,

$R_2$ represents a hydrogen atom, a straight-chained or branched alkyl group with 1 to 4 carbon atoms or an alkyl group with 2 or 3 carbon atoms substituted in the 2- or 3-position by a hydroxy or dimethylamino group,

$R_3$ represents a hydrogen atom or an alkyl group with 1 to 3 carbon atoms, and

$R_4$ represents a methyl group or a hydrogen or chlorine atom, characterised in that a compound of general formula

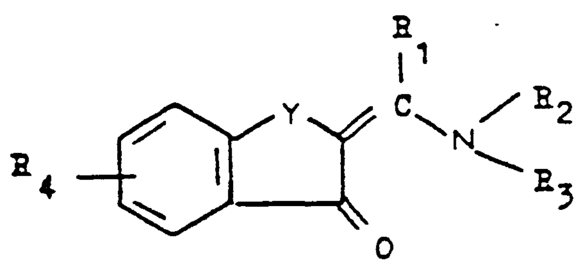

(II)

wherein

$R_1$ to $R_4$ are as hereinbefore defined and

Y represents a sulphur atom or a sulphinyl group, is oxidised.

8. Process as claimed in claim 7, characterised in that the reaction is carried out in a solvent and at temperatures of between -80 and 120°C, preferably at temperatures of between -10 and 80°C.

9. Process as claimed in claims 7 and 8 for preparing compounds of general formula I wherein X represents a sulphinyl group, characterised in that a compound of general formula II wherein Y represents a sulphur atom is oxidised with one equivalent of an oxidising agent.

10. Process as claimed in claims 7 and 8 for preparing compounds of general formula I wherein X represents a sulphonyl group, characterised in that a compound of general formula II wherein Y represents a sulphinyl group

is oxidised with one or more equivalents of an oxidising agent, whilst a compound of general formula II wherein Y represents a sulphur atom is oxidised with two or more equivalents of an oxidising agent.

**Revendications**

1. Carboxamides vinylogues de formule générale

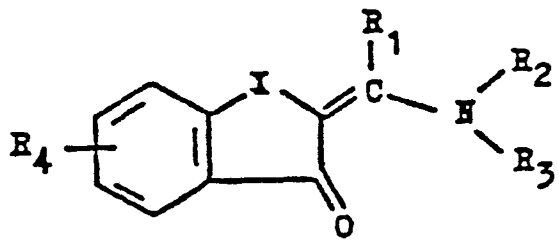

,(I)

dans laquelle
X représente le groupe sulfinyle ou sulfonyle,
$R_1$ représente un radical phényle, qui peut être substitué par des atomes d'halogène et/ou des groupes méthyle, ou représente un radical pyridinyle,
$R_2$ représente un atome d'hydrogène, un groupe alcoyle rectiligne ou ramifié avec 1 à 4 atomes de carbone ou un groupe alcoyle avec 2 ou 3 atomes de carbone, substitué en position 2 ou 3 par un groupe hydroxy ou diméthylamino,
$R_3$ représente un atome d'hydrogène ou un groupe alcoyle avec 1 à 3 atomes de carbone et
représente le groupe méthyle, un atome d'hydrogène ou de chlore.

2. Carboxamides vinylogues de formule générale I selon la revendication 1, dans laquelle
X représente le groupe sulfinyle ou sulfonyle,
$R_1$ représente un radical phényle, éventuellement substitué par un groupe méthyle, un atome de fluor, de chlore ou de brome,
$R_2$ représente un atome d'hydrogène, un groupe alcoyle avec 1 à 3 atomes de carbone ou le groupe 2-hydroxy-éthyle,
$R_3$ représente un atome d'hydrogène ou le groupe méthyle et
$R_4$ représente le groupe méthyle, un atome d'hydrogène ou de chlore.

3. Carboxamides vinylogues de formule générale I selon la revendication 1, dans laquelle
X représente le groupe sulfinyle,
$R_1$ représente le groupe phényle, 2-chlorophényle ou 2-bromophényle,
$R_2$, $R_3$ et $R_4$ représentent chacun un atome d'hydrogène.

4. Le 1-oxyde de (E)-2-[(amino)phénylméthylène]-benzo-[b]thiophène-3(2H)-one.

5. Médicament contenant un composé selon les revendications 1 à 4, éventuellement conjointement à un ou plusieurs excipients et/ou diluants inertes.

6. Utilisation d'un composé selon les revendications 1 à 4 pour la préparation d'un médicament à activité anticonvulsive.

7. Procédé pour la préparation de nouveaux carboxamides vinylogues de formule générale

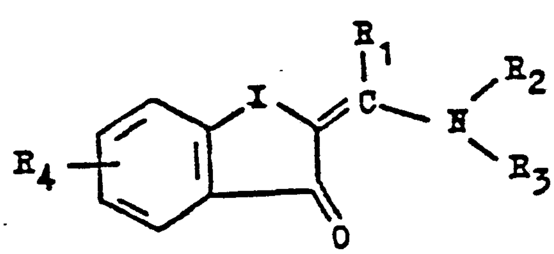

,(I)

dans laquelle
X représente le grupe sulfinyle ou sulfonyle,

$R_1$ représente un radical phényle, qui peut être substitué par des atomes d'halogène et/ou des groupes méthyle, ou représente un radical pyridinyle,

$R_2$ représente un atome d'hydrogène, un groupe alcoyle rectiligne ou ramifié avec 1 à 4 atomes de carbone ou un groupe alcoyle avec 2 ou 3 atomes de carbone substitué en position 2 ou 3 par un groupe hydroxy ou diméthylamino,

$R_3$ représente un atome d'hydrogène ou un groupe alcoyle avec 1 à 3 atomes de carbone et

$R_4$ représente le groupe méthyle, un atome d'hydrogène ou de chlore, caractérisé en ce qu'on oxyde un composé de

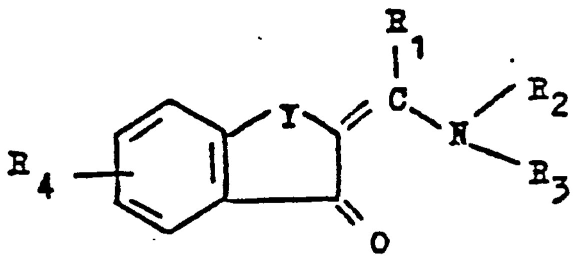

,(II)

dans laquelle

$R_1$ à $R_4$ sont définis comme au début et

Y représente un atome de soufre ou le groupe sulfinyle.

8. Procédé selon la revendication 7, caractérisé en ce que la réaction est effectuée dans un solvant et à des températures entre -80 et 120° C, de préférence cependant à des températures entre -10 et 80° C.

9. Procédé selon les revendications 7 et 8, pour la préparation de composés de formule générale I, dans laquelle X représente le groupe sulfinyle, caractérisé en ce qu'on oxyde un composé de formule générale II dans laquelle Y représente un atome de soufre, au moyen d'un équivalent d'un agent d'oxydation.

10. Procédé selon les revendications 7 et 8 pour la préparation de composés de formule générale I, dans laquelle X représente le groupe sulfonyle, caractérisé en ce qu'on oxyde un composé de formule générale II dans laquelle Y représente le groupe sulfinyle, avec un equivalent ou plus d'un agent d'oxydation, un composé de formule générale II dans laquelle Y représente un atome de soufre, avec deux équivalents ou plus d'un agent d'oxydation.